(19) **Europäisches Patentamt European Patent Office Office européen des brevets**

(11) **EP 2 739 749 B1**

(12) **EUROPEAN PATENT SPECIFICATION**

(45) Date of publication and mention
of the grant of the patent:
**21.12.2016 Bulletin 2016/51**

(51) Int Cl.:
***C12Q 1/68*** $^{(2006.01)}$

(21) Application number: **12742926.4**

(86) International application number:
**PCT/EP2012/065376**

(22) Date of filing: **06.08.2012**

(87) International publication number:
**WO 2013/017701 (07.02.2013 Gazette 2013/06)**

(54) **METHYLATION SIGNATURE FOR REPLICATIVE SENESCENCE OF CELLS IN CULTURE**

METHYLIERUNGSSIGNATUR ZUR REPLIKATIVEN SENESZENZ VON ZELLEN IN KULTUR

SIGNATURE DE MÉTHYLATION POUR LA SÉNESCENCE RÉPLICATIVE DES CELLULES EN CULTURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.08.2011 EP 11176593**

(43) Date of publication of application:
**11.06.2014 Bulletin 2014/24**

(73) Proprietor: **Rheinisch-Westfälische Technische Hochschule Aachen**
**52062 Aachen (DE)**

(72) Inventors:
 • **WAGNER, Wolfgang**
  **52078 Aachen (DE)**
 • **KOCH, Carmen**
  **52066 Aachen (DE)**
 • **JOUSSEN, Sylvia**
  **52249 Eschweiler (DE)**
 • **SCHELLENBERG, Anne**
  **52064 Aachen (DE)**

(74) Representative: **Remus, Alvaro Johannes**
**BPSH Schrooten Haber Remus**
**Patent- und Rechtsanwaltspartnerschaft mbB**
**Mörsenbroicher Weg 191**
**40470 Düsseldorf (DE)**

(56) References cited:
 • BORK, S. ET AL.: "DNA Methylation Pattern Changes upon Long-Term Culture and Aging of Human Mesenchymal Stromal Cells", AGING CELL, vol. 9, 6 November 2009 (2009-11-06), pages 54-63, XP002663876, cited in the application
 • WAGNER WOLFGANG ET AL.: "How to track cellular aging of mesenchymal stromal cells?", AGING, NEW YORK, NY, US, vol. 2, no. 4, 1 April 2010 (2010-04-01), pages 224-230, XP002663877, ISSN: 0160-2721 [retrieved on 2010-04-08]
 • KOCH, C. ET AL.: "Specific Age-associated DNA Methylation Changes in Human Dermal Fibroblasts", PLOS ONE, vol. 6, 2011, page E16679, XP002663878, cited in the application
 • KOTARO R. SHIBATA ET AL: "Expression of the p16INK4A Gene Is Associated Closely with Senescence of Human Mesenchymal Stem Cells and Is Potentially Silenced by DNA Methylation During In Vitro Expansion", STEM CELLS, vol. 25, no. 9, 1 September 2007 (2007-09-01), pages 2371-2382, XP055012510, ISSN: 1066-5099, DOI: 10.1634/stemcells.2007-0225
 • RUSH L J ET AL: "Epigenetic profiling in chronic lymphocytic leukemia reveals novel methylation targets", CANCER RESEARCH, AMERICAN ASSOCIATION FOR CANCER RESEARCH, US, vol. 64, 1 January 2004 (2004-01-01), pages 2424-2433, XP003023713, ISSN: 0008-5472, DOI: 10.1158/0008-5472.CAN-03-2870
 • "Illumina HumanMethylation27 BeadChip", GEO; NCBI, 27 April 2009 (2009-04-27), XP002663879,

• BOCKLANDT S ET AL: 'Epigenetic Predictor of Age' PLOS ONE, PUBLIC LIBRARY OF SCIENCE, US vol. 6, no. 6, E14821, 22 June 2011, pages 1 - 6, XP002687317 DOI: 10.1371/JOURNAL.PONE.0014821 ISSN: 1932-6203

Remarks:
The complete document including Reference Tables and the Sequence Listing can be downloaded from the EPO website

**Description**

BACKGROUND OF THE INVENTION

The invention relates to DNA-methylation and its association with cellular aging. In particular, the invention concerns a method and the use of a kit for determining the replicative senescence status of a cell, wherein the methylation status of at least one of the CpG-dinucleotide is determined and compared with a reference methylation status of the respective CpG-dinucleotide.

Cell therapy and tissue engineering raise tremendous expectations with respect to future clinical applications. This is for example reflected by an increasing number of clinical studies in this technical field. Mesenchymal stromal cells (MSCs) are of particular interest for clinical applications, because they can be isolated from a variety of tissues, and they comprise a rare population of adult stem cells having the potential of multi-lineage differentiation.

PRIOR ART

For their clinical use, it is necessary to isolate cells from at least one of a variety of tissues, for example from dermis, bone marrow or adipose tissue, and to generate a sufficiently large number of cells by expansion *in-vitro,* i.e. cell culture. However, mammalian cells can be expanded in culture for a limited number of passages only. The cells enter a senescent state and stop proliferation. Notably, this phenomenon is not observed in cultures of embryonic stem cells and induced pluripotent stem cells as long as these cells maintain their totipotent or pluripotent state. This difference indicates that this phenomenon is based on a regulated aging process rather than a mere accumulation of cellular defects. Cellular aging is associated with increasing cell size and a flattened cellular morphology. The proliferation rate of the cell and *in vitro* differentiation of various cell types such as MSCs declines with increasing numbers of cell passages. Moreover, cells acquire mutations in long-term culture which may result in malignant transformation of affected cells.

The alteration of cellular morphology and functionality during cellular aging has a tremendous impact on the engraftment and success of transplantation when cells are used in clinical therapy. Therefore, it is essential to consider cellular aging and the age of cells as a measure for their quality, especially in the emerging field of cellular therapy. However, no standards for expanding MSCs *in vitro* and their use in therapy are available yet. This may be due to the lack of standardized conditions for their isolation and the lack of specific molecular markers.

Commonly used parameters for assessing cellular aging are passage number, cumulative population doubling, and time of *in vitro* culture. However, these parameters have to be documented very thoroughly throughout culture expansion of the cells. Otherwise it would be impossible to determine the cellular age. Karyotyping and SNP-arrays are recommended for analyzing cells in culture. But these methods are not suitable for finding a malignant subclone in a heterogeneous mixture of cells. Expression of senescence-associated beta-galactosidase can discern cells at their senescent stage, but hardly provides a quantitative measure for cellular aging. Cellular senescence is clearly associated with a loss of telomere integrity, and initial telomere length has been shown to correlate with replicative capacity. However, telomere length and telomerase expression varies in different cell types and have not proven as reliable quantitative measure for cellular aging.

Therefore, there is a need for molecular markers which allow a reliable assessment of the replicative senecscense of cells. Recently, it had been discovered that long-term culture of MSCs or fibroblasts is associated with specific epigenetic modifications in DNA-methylation profiles (Bork, S. et al. DNA Methylation Pattern Changes upon Long-Term Culture and Aging of Human Mesenchymal Stromal Cells. Aging Cell 9, 54-63 (2010); Koch, C. et al. Specific Age-associated DNA Methylation Changes in Human Dermal Fibroblasts. PLoS ONE 6, e16679 (2011). It was discovered that the methylation pattern of a variety of genes differed in cells from early passages (P2) and late passages (P8 to P 15) in that the methylation status at 27,578 CpG dinucleotides was simultaneously analyzed using a microarray.

It is however necessary to provide markers which are more precise and reliable in assessing the replicative senescence of cells, and which may not only allow for distinguishing cells of an early passage from cells of a late passage, but which show a clear and unequivocal correlation between methylation status and passage number.

SUMMARY OF THE INVENTION

The present invention provides a method for determining the replicative senescence status of a cell propagated in an *in-vitro* culture. The method comprises the steps of determining the methylation status of least one of the CpG-dinucleotides within a region of about 10,000 bp upstream and/or downstream of at least one of the CpG-dinucleotides selected from the group consisting of GRM7-CpG-site #1 (SEQ ID No. 33), CASR-CpG-site #1 (SEQ ID No.34), PRAMEF2-CpG-site #1 (SEQ ID No. 35), SELP-CpG-site #1 (SEQ ID No. 36), CASP14-CpG-site #1 (SEQ ID No. 37) and KRTAP13-3-CpG-site #1 (SEQ ID No. 38), and of comparing the methylation status of the CpG-dinucleotide(s) with a reference methylation status for each of the respective CpG-dinucleotide(s), wherein the methylation status of said CpG-dinucleotide linearly correlates with the replicative senescence status of said cell. By comparing the methylation status of each of the at least one CpG-dinucleotides investigated with a reference methylation status of the respective CpG-dinucleotide, the replicative senescence status is determined. The term methylation status refers to the level of methylation, i.e. the number of methylated versus non-methylated CpG-dinucleotides of a specific GpG-dinucleotide. Thus, it is preferred that multiple cells are analysed for their methylation status. Thus, the methylation status of least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 of multiple cells, i.e. for multiple corresponding DNA molecules is determined, and compared with the reference methylation status of the respective CpG-dinucleotide, whereby the replicative senescence status is determined. The reference methylation status for a specific CpG-dinucleotide is preferably an empirically determined methylation level representing a correlation between the methylation level of said CpG-dinucleotide and one or more of the duration cells spent in *in-vitro* culture, the number of passages cells went through, the cumulative population doubling and the days of *in-vitro* culture of the cells. The CpG-dinucleotides within a region of about 10,000 bp upstream and/or downstream of at least one of the CpG-dinucleotides selected from the group consisting of GRM7-CpG-site #1 (SEQ ID No. 33), CASR-CpG-site #1 (SEQ ID No.34), PRAMEF2-CpG-site #1 (SEQ ID No. 35), SELP-CpG-site #1 (SEQ ID No. 36), CASP14-CpG-site #1 (SEQ ID No. 37) and KRTAP13-3-CpG-site #1 (SEQ ID No. 38) are continuously methylated or demethylated with increasing senescence of the cells, i.e. a straight proportional change of the methylation status of these CpG-dinucleotides can be observed during proceeding senescence of the cells.

The CpG-dinucleotides within a region of about 10,000 bp upstream and/or downstream of at least one of the CpG-dinucleotides selected from the group consisting of GRM7-CpG-site #1 (SEQ ID No. 33), CASR-CpG-site #1 (SEQ ID No.34), PRAMEF2-CpG-site #1 (SEQ ID No. 35), SELP-CpG-site #1 (SEQ ID No. 36), CASP14-CpG-site #1 (SEQ ID No. 37) and KRTAP13-3-CpG-site #1 (SEQ ID No. 38) are all suitable to determine the senescence status of different cell populations (e.g. fibroblasts or MSCs cultured under various conditions and with different methods).

In a preferred embodiment of the invention, the methylation status of at least one of the CpG-dinucleotides within a region of about 5,000 bp upstream and/or downstream, even more preferably of about 3,000 bp upstream and/or downstream, even more preferably of about 1,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 is determined.

In another preferred embodiment of the invention, the replicative senescence status is determined in that the methylation status is determined for at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules.

In another preferred embodiment of the invention, the cell has been freshly isolated from a donor.

In another preferred embodiment of the invention, the cell has been cultured for some time.

In another preferred embodiment of the invention, the cell is selected from the group consisting of stromal cells and induced pluripotent stem cells.

In another preferred embodiment of the invention, the methylation status of two, three, four, five or all of said six CpG-dinucleotides from different DNA molecules which are selected from the group consisting of DNA molecules comprising at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 is determined.

In another preferred embodiment of the invention, the methylation status is determined by one or more methods selected from the group consisting of methylation specific PCR, COBRA-Assay, methylation-specific restriction pattern analysis, CHIP-sequencing, methyl-CAP-sequencing, and preferably sequence analysis of bisulfite-treated DNA.

The invention further includes the use of at least one nucleic acid molecule for determining the replicative senescence status of a cell according to the method according to the invention, wherein said nucleic acid molecule comprises at least one nucleotide sequence selected from the group consisting of:

a) a nucleotide sequence comprising at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1,
b) a nucleotide sequence which differs from the nucleotide sequence of a) by replacement of at most 10 % of the nucleotides, but for said CpG-dinucleotide, and
c) a nucleotide sequence which corresponds to the complementary strand of the nucleotide sequence of a) or b).

In a preferred embodiment of this use, at least one of the CpG-dinucleotides within a region of about 5,000 bp upstream and/or downstream, even more preferably of about 3,000 bp upstream and/or downstream, even more preferably of about 1,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 is used.

In another preferred embodiment of this use, at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

The invention further includes the use of a kit in the method according to the invention, the kit comprises at least one set of oligonucleotide primers for amplifying and/or analyzing at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

In a preferred embodiment, this use of a kit comprises at least one oligonucleotide primer for amplifying and/or analyzing at least one of the CpG-dinucleotides within a region of about 5,000 bp upstream and/or downstream, even more preferably of about 3,000 bp upstream and/or downstream, even more preferably of about 1,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

In another preferred embodiment, this use of a kit comprises a pair of oligonucleotide primers for amplifying and/or at least one oligonucleotide primer for analyzing at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

In a particularly preferred embodiment, said at least one oligonucleotide primer is selected from the group consisting of SEQ ID NO: 7 to SEQ ID NO: 24.

In another preferred embodiment, this use of a kit further comprises at least one reaction buffer and/or reagents for at least one method selected from the group consisting of PCR-amplification, bisulfite-conversion of DNA, DNA-sequencing, preferably DNA-pyrosequencing, and COBRA-assay.

According to another advantageous aspect of the invention, a computer-readable medium is provided, which has stored computer-executable instructions for causing a computer to perform a method for determining the replicative senescence status of a cell according to the method of any one of claims 1 to 7 comprising:

- inputting at least one value of the determined methylation status of at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1,
- comparing said value of the determined methylation status with stored data representing a correlation between the methylation status of said CpG-dinucleotide and the replicative senescence status of the cell, and
- displaying the replicative senescence status of the cell.

In a particularly preferred embodiment of the invention, said stored data comprise at least one linear regression equation.

The term "replicative senescence status" refers to the potential of a cell to undergo further cell divisions and to differentiate into different cell types. The replicative senescence status of a cell depends on the duration of the cell in *in-vitro* culture, on the culture conditions, on the number of passages, and on the age of the donor. A cell having a less advanced replicative senescence status has a higher differentiation potential and a higher proliferation potential then a cell having a more advanced replicative senescence status. Hence, when referring to a method to determine the replicative senescence status of a cell, it is intended and suitable for determining any one or more of the duration said cells spent in *in-vitro* culture, the number of passages said cells went through, the cumulative population doubling and the days of *in-vitro* culture of said cells.

These and other aspects of the invention will be apparent from and elucidated with reference to the exemplary embodiments and figures described hereinafter.

BRIEF DESCRIPTION OF THE DRAWINGS

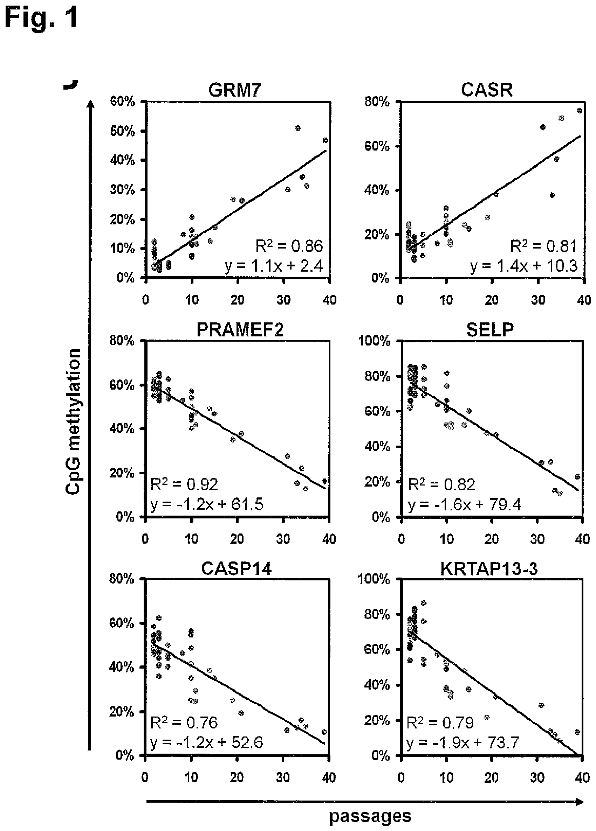

Fig. 1    depicts the alteration in methylation of specific genes in relation to the number of passages of the cells *in-vitro*.

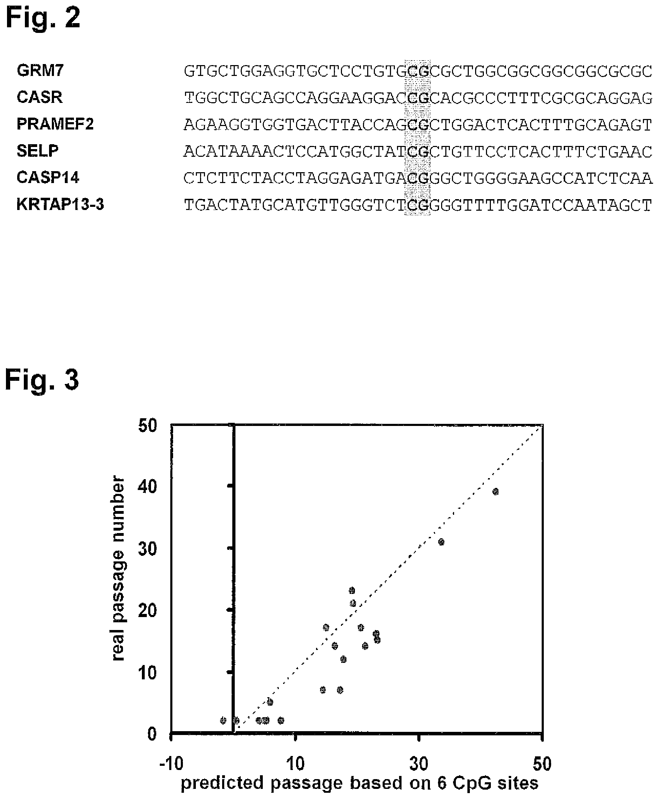

Fig. 2    shows the GRM7-CpG-site #1, the CASR-CpG-site #1, the PRAMEF2-CpG-site #1, the SELP-CpG-site #1, the CASP14-CpG-site #1 and the KRTAP13-3-CpG-site #1, as well as the adjacent 20 nucleotides upstream and downstream of each of said CpG-sites.

Fig. 3 illustrates the correlation of predicted passage numbers by means of the methylation status of six CpG-dinucleotides with the real passage number of the cells *in-vitro.*

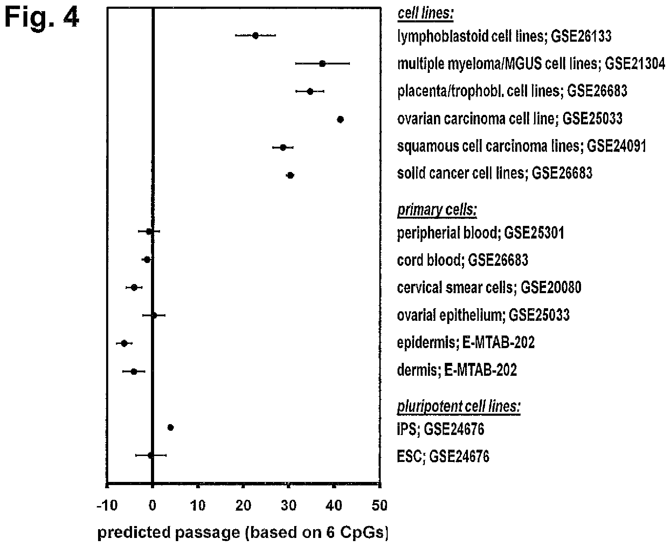

Fig. 4 illustrates the predicted passage number of a variety of primary cells and a variety of cell lines.

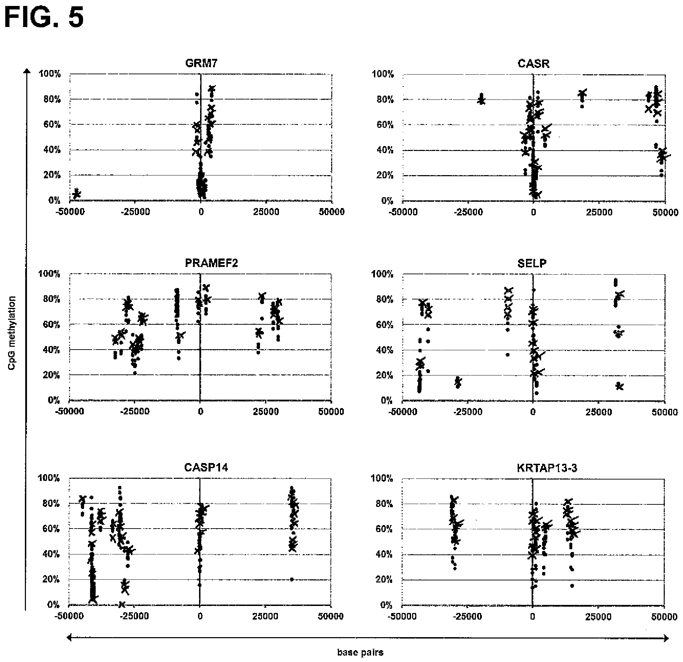

Fig. 5 shows the methylation status of CpG-dinucleotides within a region of 50,000 bp upstream and downstream of the GRM7-CpG-site #1, the CASR-CpG-site #1, the PRAMEF2-CpG-site #1, the SELP-CpG-site #1, the CASP14-CpG-site #1 and the KRTAP13-3-CpG-site #1 (respectively referred to as "0") of human bone marrow cells (hBMCs); each x represents the methylation status of an early passage, each dot represents the methylation status of a later (presenescent) passage.

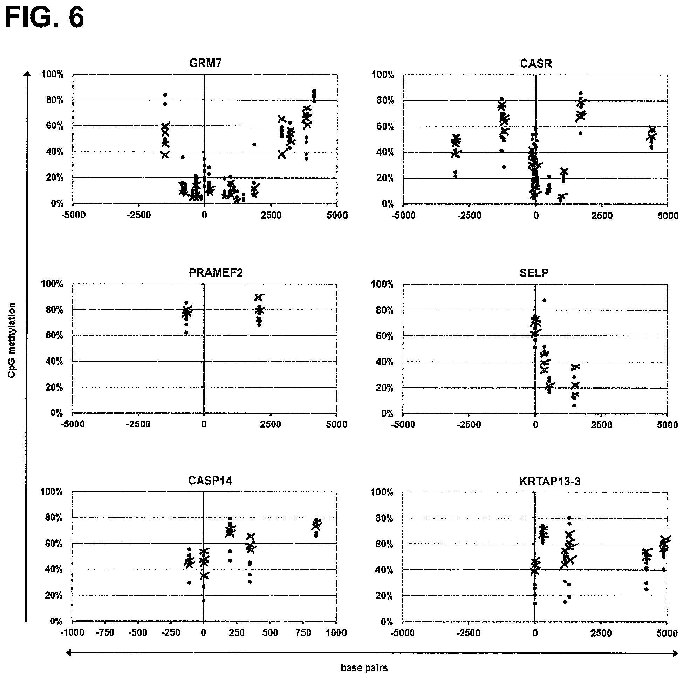

Fig. 6 shows the methylation status of CpG-dinucleotides within a region of 5,000 bp upstream and downstream of the GRM7-CpG-site #1, the CASR-CpG-site #1, the PRAMEF2-CpG-site #1 (methylation data of this site were not available on the Illumina 450k chip used here), the SELP-CpG-site #1, and the KRTAP13-3-CpG-site #1 (respectively referred to as "0") and within a region of 1,000 bp upstream and downstream of the CASP14-CpG-site #1 (also referred to as "0") of human bone marrow cells (hBMCs), each x represents the methylation status of an early passage, each dot represents the methylation status of a later (presenescent) passage.

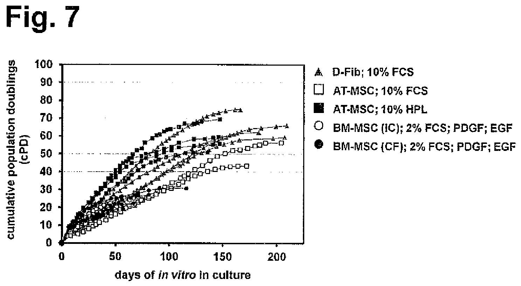

Fig. 7 shows long-term growth curves of culture-expanded cells. Human dermal fibroblasts and mesenchymal stem cells from bone marrow and adipose tissue were expanded in vitro using the indicated supplements. Long-term growth curves of the training data are based on the documentation of culture time and calculation of cumulative population doublings.

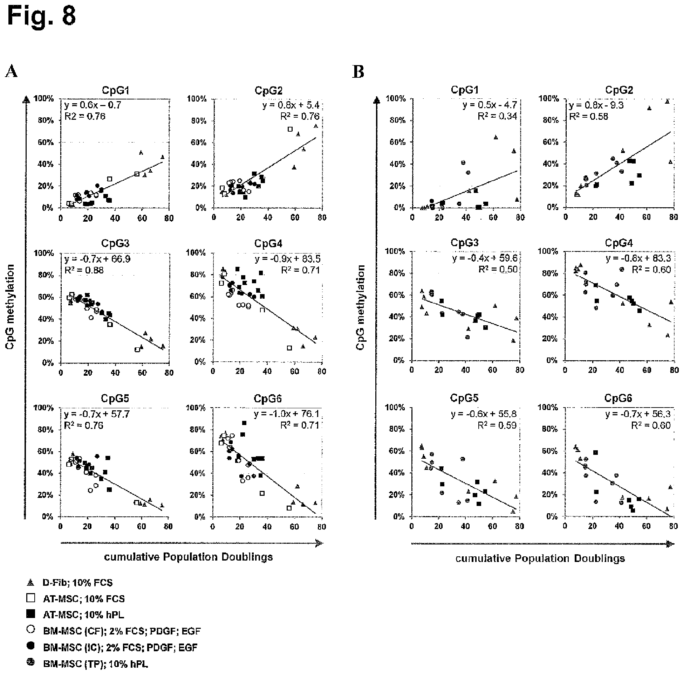

Fig. 8 shows Cumulative Population Doublings (cPD) reflected by the DNA methylation level of CpG sites GRM7-CpG-site #1 (CpG1), CASR-CpG-site #1 (CpG2), PRAMEF2-CpG-site #1 (CpG3), SELP-CpG-site #1 (CpG4), CASP14-CpG-site #1 (CpG5) and KRTAP13-3-CpG-site #1 (CpG6). The DNA methylation levels of these 6 specific CpG sites are plotted against the calculated cumulative Population Doublings of the training **(A)** and validation data **(B).** A clear correlation between cPD and methylation status is visible for both datasets. Regression lines, equations and regression coefficients are provided in the figure as well as media and supplements.

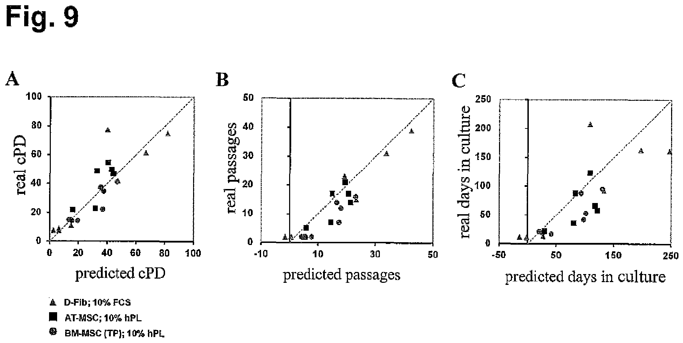

Fig. 9 represents predictions of cPD, passage number and days in culture based on the Senescence Signature according to the invention. Linear regression models on the basis of DNA methylation at the 6 specific CpG sites (GRM7-CpG-site #1, CASR-CpG-site #1, PRAMEF2-CpG-site #1, SELP-CpG-site #1, CASP14-CpG-site #1 and KRTAP13-3-CpG-site #1) are applied to calculate cPD **(A),** passage numbers **(B)** or days in culture **(C)** of the validation data. These predictions are plotted against real cPD, passage numbers and days in culture. Regression coefficients are given in the figure.

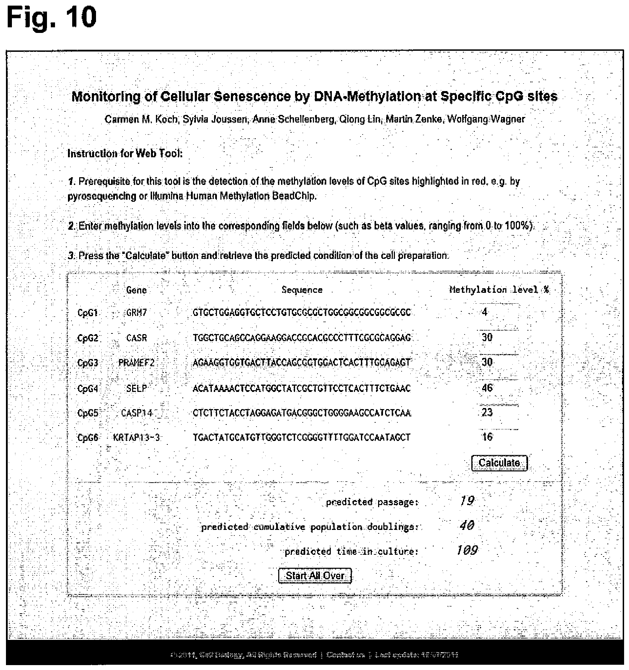

Fig. 10 shows a screenshot of an input mask of a software designed for automated prediction of cPD, passages and days in culture of a given cell preparation. After typing in the corresponding methylation beta-values of the 6 specific CpG sites (GRM7-CpG-site #1, CASR-CpG-site #1, PRAMEF2-CpG-site #1, SELP-CpG-site #1, CASP14-CpG-site #1 and KRTAP13-3-CpG-site #1) and pressing the *Calculate* button, the results are calculated and displayed.

## DESCRIPTION OF EXEMPLARY AND PREFERRED EMBODIMENTS OF THE INVENTION

According to a first aspect, the present invention provides a method for determining the replicative senescence status of a cell. The method comprises the steps of determining the methylation status, i.e. the status of methylated versus non-methylated versions of corresponding DNA molecules, by determining the methylation status of least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules, and of comparing the methylation status of each of the at least one CpG-dinucleotides investigated with a reference methylation status of the respective CpG-dinucleotide, whereby the replicative senescence status is determined.

In a preferred embodiment, the method according to the first aspect comprises determining the methylation status by determining the methylation status of least one of the CpG-dinucleotides within a region of about 5,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules. More preferably, the method comprises determining the methylation status by determining the methylation status of at least one of the CpG-dinucleotides within a region of about 3,000 bp upstream and/or downstream, of about 1,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules. Even more preferably, the method comprises determining the methylation status by determining the methylation status of least one of the CpG-dinucleotides selected from the group consisting of the CpG-dinucleotides of the GRM7 gene, the CASR gene, the PRAMEF2 gene, the SELP gene, the CASP14 gene, and the KRTAP13-3 gene. Most preferably, the method comprises determining the methylation status by determining the methylation status of least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

The region of about 100,000 bp upstream and downstream of CpG-dinucleotide GRM7-CpG-site #1 is represented by SEQ ID No. 33. The region of about 100,000 bp upstream and downstream of CpG-dinucleotide CASR-CpG-site #1 is represented by SEQ ID No. 34. The region of about 100,000 bp upstream and downstream of CpG-dinucleotide PRAMEF2-CpG-site #1 is represented by SEQ ID No. 35. The region of about 100,000 bp upstream and downstream of CpG-dinucleotide SELP-CpG-site #1 is represented by SEQ ID No. 36. The region of about 100,000 bp upstream and downstream of CpG-dinucleotide CASP14-CpG-site #1 is represented by SEQ ID No. 37. The region of about 100,000 bp upstream and downstream of CpG-dinucleotide KRTAP13-3-CpG-site #1 is represented by SEQ ID No. 38. It is understood that the respective SEQ ID Nos: 33 to 38 also include the regions of about 80,000 bb, 60,000 bp, 50,000 bp, 40,000 bg, 30,000 bp, 20,000 bp, 10,000 bp, 5,000 bp, 3,000 bp, and 1,000 bp upstream and downstream of each of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

The cells may be cells that have been freshly isolated from a donor. This embodiment may for example permit monitoring the replicative senescence status of cells that were previously implanted for therapeutic use. It is possible to distinguish between native cells of the patient and foreign or native cells that were implanted, i.e. cultured for some time after being isolated from their donor, and before being implanted into the patient. In another embodiment of the method according to the first aspect, the cells may have been cultured *in-vitro* for some time. This embodiment of the method allows determining the approximate time, said cells have been in culture, and/or the approximate passage number of said cells and/or the number of population doublings. In a preferred embodiment of the method, the cells are mesenchymal stromal cells.

In another embodiment, the cells are induced pluripotent stem cells. Determining the replicative senescence status for induced pluripotent stem cell can be used to quantify the reprogramming efficiency.

In yet an additional or alternative embodiment, the cells may have been frozen at any time and for any period of time before being analysed. This embodiment is of particular relevance when retained samples of cells that were implanted for therapeutic use are to be analysed for their replicative senescent status prior to being implanted.

The replicative senescence status of a cell can be determined by determining the methylation status of a single CpG-dinucleotides within a region of about 10,000 bp upstream and/or downstream of at least one of the CpG-dinucleotides, selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules. Preferably the methylation status of two, three, four, five six different CpG-dinucleotides is determined, wherein said two, three, four, five or six CpG-dinucleotides are chosen from different DNA molecules of the group consisting of DNA molecules comprising GRM7-CpG-site #1, CASR-CpG-site #1, PRAMEF2-CpG-site #1, SELP-CpG-site #1, CASP14-CpG-site #1 and KRTAP13-3-CpG-site #1. It is even more preferred that the methylation status of two, three, four, five or all six different CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 is determined for multiple corresponding DNA. The more genes of said group will be involved in determining their methylation status, the more precise the determination of their replicative senescence status.

In embodiments of the method according to the first aspect, the methylation status is determined by one or more suitable methods selected from - but not restricted to - the group consisting of methylation specific PCR, sequence analysis of bisulfite-treated DNA, COBRA-Assay, CHIP-Sequencing, Next-Generation Sequencing, Methyl-CAP-sequencing and methylation-specific restriction patterns. MSP can rapidly assess the methylation status of virtually any group of CpG dinucleotides within a CpG island, independent of the use of cloning or methylation-sensitive restriction enzymes. The MSP comprises initial modification of DNA by sodium bisulfite, converting all unmethylated, but not methylated, cytosines to uracil, and subsequent amplification with primers specific for methylated versus unmethylated DNA.

MSP requires very small quantities of DNA, and is sensitive to 0.1% methylated alleles of a given CpG island locus. Preferably, the methylation status is determined by pyrosequencing of bisulfite-treated DNA which allows identifying whether a specific CpG-dinucleotide was methylated or not, and thereby provides an accurate value for the percentage of methylated CpG-dinucleotides of a given CpG-dinucleotide.

The COBRA assay is a quantitative technique to determine DNA methylation levels at specific gene loci in small amounts of genomic DNA. In the COBRA assay, restriction enzyme digestion is used to reveal methylation dependent sequence differences in PCR products of sodium bisulfite-treated DNA. Methylation levels in the original DNA sample are represented by the relative amounts of digested and undigested PCR product in a linearly quantitative fashion across a wide spectrum of DNA methylation levels. The COBRA assay is easy to use and provides quantitative accuracy.

In the COBRA assay, methylation-dependent sequence differences are introduced into the genomic DNA by sodium bisulfite treatment and subsequent PCR amplification of the bisulfite treated DNA. This combination of bisulfite treatment and PCR amplification results in conversion of unmethylated cytosine residues to thymine and of methylated cytosine residues to cytosine. This sequence conversion can lead to methylation dependent creation of new restriction enzyme sites or it can lead to the methylation dependent retention of pre-existing restriction enzyme sites such as, for example, *Bst*UI (CGCG). The primers used in the PCR amplification reaction do not contain CpG dinucleotides so that the amplification step does not discriminate between templates according to their original methylation status. Therefore, in the mixed population of DNA fragments resulting from said PCR, the fraction that has a newly created or retained restriction site that contains a CpG(s) directly reflects the percentage of DNA methylation at that site in the original genomic DNA.

Notwithstanding that virtually any method for analyzing the methylation status of a given CpG-dinucleotide can be employed for analyzing the methylation status of at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1, it is preferred that the methylation status is determined by direct sequence analysis of bisulfite-treated DNA.

According to an embodiment of the invention, the methylation status of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1is determined by pyrosequencing of bisulfite-treated DNA.

In animals DNA methylation predominantly involves the addition of a methyl group to the carbon-5 position of cytosine residues of the dinucleotide CpG, and is implicated in repression of transcriptional activity. Treatment of DNA with sodium bisulfite converts cytosine residues to uracil, but leaves 5-methylcytosine residues unaffected. Thus, sodium bisulfite treatment introduces specific changes in the DNA sequence that depend on the methylation status of individual cytosine residues, yielding single-nucleotide resolution information about the methylation status of a segment of DNA.

According to another or additional embodiment of the invention, methylation of the at least one CpG-dinucleotide is determined by sequence analysis of bisulfite treated DNA.

The method of methylation analysis utilizing sequence analysis of bisulfite-treated DNA involves bisulfite treatment of the DNA to be analyzed, PCR amplification of the bisulfite treated DNA, cloning of the amplification product, and standard dideoxynucleotide DNA sequencing of the cloned DNA fragments to directly determine the nucleotides resistant to bisulfite conversion. Preferred primers for PCR amplification are designed to be strand-specific but not methylation-specific, i.e. the nucleotide sequence of the preferred primers do not comprise a sequence corresponding to a nucleotide sequence including a CpG dinucleotide. Hence, the preferred primers for PCR amplification flank but do not involve the methylation site or methylation sites of interest. However, in an additional or alternative embodiment, at least one or both, the forward primer and the reverse primer for PCR amplification may cover one or more CpG-dinucleotides. To be strand specific and to allow amplification of methylated as well as non-methylated DNA fragments, a mixture of primers may be utilized, wherein the mixture of strand primers consists of primers having a pyrimidine nucleotide (Y) for the cytosine of the CpG dinucleotide within the DNA fragment to be amplified and covered by said strand primer, i. e. a C for amplification of said DNA fragment which is methylated at said C, or a T for amplification of said DNA fragment which is not methylated at said C. Therefore, the PCR amplification will amplify both methylated and unmethylated sequences, in contrast to methylation-specific PCR. All sites of unmethylated cytosines are displayed as thymines in the resulting amplified sequence of the sense strand, and as adenines in the amplified antisense strand. This method requires cloning of the PCR products prior to sequencing for adequate sensitivity. Alternatively, nested PCR methods can be used to enhance the product for sequencing.

Pyrosequencing may also be used to analyze bisulfite-treated DNA without using methylation-specific PCR. Following PCR amplification of the region of interest, pyrosequencing is used to determine the bisulfite-converted sequence of specific CpG sites in the region. The status of C-to-T at individual sites can be determined quantitatively based on the amount of C and T incorporation during the sequence extension.

According to another or additional embodiment of the invention, methylation of the at least one of the genes selected from the group consisting of GRM7, CASR, PRAMEF2, SELP, CASP14 and KRTAP13-3 is determined by combined bisulfite restriction analysis (COBRA assay) where the COBRA-assay can be employed.

GRM7 denotes the human metabotropic glutamate receptor gene, namely the gene for the isoform b precursor which is located on chromosome 3 at p26.1. GRM7 is registered under gene ID: 2917 in the NCBI Gene database (http://www.ncbi.nlm.nih.gov/gene/2917). The first GRM7 transcript variant (SEQ ID No. 25) is registered in the NCBI Gene Bank database under accession no. NM_000844. The second GRM7 transcript variant (SEQ ID NO: 26) is registered in the NCBI Gene Bank database under accession no. NM_181874.

CASR denotes the human calcium-sensing receptor gene which is located on chromosome 3 at q21.1. CASR is registered under gene ID: 846 in the NCBI Gene database (http://www.ncbi.nlm.nih.gov/gene/846). The first CASR transcript variant (SEQ ID No. 27) is registered in the NCBI Gene Bank database under accession no. NM_001178065. The CASR second transcript variant (SEQ ID NO: 28) is registered in the NCBI Gene Bank database under accession no. NM_000388.

PRAMEF2 denotes the human gene for PRAME family member 2 which is located on chromosome 1 at p36.21. PRAMEF2 is registered under gene ID: 65122 in the NCBI Gene database (http://www.ncbi.nlm.nih.gov/gene/65122). The PRAMEF2 transcript variant (SEQ ID No. 29) is registered in the NCBI Gene Bank database under accession no. NM_023014.

SELP denotes the human selectin P precursor gene which is located on chromosome 1 at q24.2. SELP is registered under gene ID: 6403 in the NCBI Gene database (http://www.ncbi.nlm.nih.gov/gene/6403). The SELP transcript (SEQ ID No. 30) is registered in the NCBI Gene Bank database under accession no. NM_003005.

CASP14 denotes the human gene encoding the caspase 14 precursor. This gene is located on chromosome 19 at p13.12. CASP14 is registered under gene ID: 23581 in the NCBI Gene database (http://www.ncbi.nlm.nih.gov/gene/23581). The CASP14 transcript (SEQ ID No. 31) is registered in the NCBI Gene Bank database under accession no. NM_012114.

KRTAP13-3 denotes the human gene for the keratin associated protein 13-3, the gene being located on chromosome 21 at q22.11. KRTAP13-3 is registered under gene ID: 337960 in the NCBI Gene database (http://www.ncbi.nlm.nih.gov/gene/337960). The KRTAP13-3 transcript (SEQ ID No. 32) is registered in the NCBI Gene Bank database under accession no. NM_181622.

According to a further aspect, the invention provides the use of a kit-of-parts (kit) for determining the replicative senescence status of a cell. The kit for determining the replicative senescence status of a cell contains means for determining the methylation status of at least one of the CpG-dinucleotides within a region of about 10,000 basepairs (bp) upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules.

In preferred embodiments, the kit comprises parts rendering the kit suitable for determining the methylation status by determining the methylation status of least one of the CpG-dinucleotides within a region of about 5,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules. More preferably, the kit comprises parts for determining the methylation status by determining the methylation status of at least one of the CpG-dinucleotides within a region of about 3,000 bp upstream and/or downstream, of about 1,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules. Even more preferably, the kit comprises parts for determining the methylation status by determining the methylation status of at least one of the CpG-dinucleotides selected from the group consisting of the CpG-dinucleotides of the GRM7 gene, the CASR gene, the PRAMEF2 gene, the SELP gene, the CASP14 gene, and the KRTAP13-3 gene. Most preferably, the kits comprise parts for determining the methylation status by determining the methylation status of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

In a particularly preferred embodiment, said kit comprises at least one oligonucleotide primer for analyzing at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1, said at least one oligonucleotide primer being adapted for amplifying and/or analyzing a nucleic acid molecule comprising at least one of the nucleotide sequences selected from the group consisting of SEQ ID NO: 1 to SEQ ID NO: 6.

In preferred embodiments of the kit, said kit comprises at least one oligonucleotide primer selected from the group consisting of SEQ ID NO: 7 to SEQ ID NO: 24.

In other or additional embodiments, the kit may further comprise at least one reaction buffer and/or reagents for at least one method selected from the group consisting of PCR-amplification, bisulfite-conversion of DNA, DNA-sequencing, preferably DNA-pyrosequencing, Next-Generation-Sequencing and COBRA-assay. These embodiments provide the advantage that all or at least almost all buffers and reagents that are necessary for determining the methylation status are provided with the kit.

While the invention has been illustrated and described in detail in the drawings and foregoing description, such illustration and description are to be considered illustrative or exemplary and not restrictive; the invention is not limited to the disclosed embodiments.

Other variations to be disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting scope.

## EXAMPLE 1

Isolation of primary cells

All samples were taken after written consent and have been specifically approved by local ethic committees on the use of human subjects. Fibroblasts were isolated from dermis (permit number of ethics committee: #EK173/07, Aachen). MSC-AT were isolated from subcutaneous adipose tissue derived from surgical interventions (#EK173/07, Aachen). MSC-BM were isolated from bone marrow aspirations from the iliac crest of healthy donors for allogeneic transplantation or from the caput femoris after hip fracture (#EK128/00, Aachen; #076/2007 and #348/2004, Heidelberg).

Culture conditions and long-term growth curves

Standard culture medium consisted of DMEM medium (PAA, Cölbe, Germany; 1g/L glucose) with L-glutamine (PAA), penicillin/streptomycin (PAA) supplemented with either 10% fetal calf serum (FCS; Biochrom, Berlin, Germany) or 10% human platelet lysate. Alternatively we used a medium consisting of 58% DMEM-LG (Cambrex, Apen, Germany), 40% MCDB201 (Sigma, Deisenhofen, Germany), 2% FCS (Stemcell Technologies, Vancouver, Canada), 2 mM L-glutamine, 100 U/ml Pen/Strep (Gibco, - Eggenstein, Germany), 1% insulin transferrin selenium, 1% linoleic acid bovine serum albumin, 10 nM dexamethasone, 0.1 mM L-ascorbic-acid-2-phosphate (all from Sigma) supplemented with platelet derived growth factor (PDGF) and epidermal growth factor (EGF; both 10 ng/ml, R&D Systems, Wiesbaden, Germany) as described before.

Cells were always harvested by trypsinisation upon 80% confluent growth, counted with a Neubauer chamber (Brand, Wertheim, Germany) or with a CASY cell counter (Schärfe System, Reutlingen, Germany) and re-seeded at a density of either 5,000 cells/cm$^2$ (fibroblasts and MSC-AT) or 10,000 cells/cm$^2$ (MSC-AT and MSC-BM). Cell Population doublings per passage (PDP) and cumulative population doublings (cPD) were calculated as described before.

Quality control of cell preparations

Surface marker expression was analyzed on a FACS canto II (Becton Dickinson Biosciences [BD], Heidelberg, Germany) upon staining with the following antibodies as described before: CD14-allophycocyanin (APC, clone M5E2, BD), CD29-phycoerythrin (PE, clone MAR4, BD), CD31-PE (clone WM59, BD), CD34-APC (clone 8G12, BD), CD45-APC (clone HI30, BD), CD73-PE (clone AD2, BD), CD90-APC (clone 5E10, BD) and CD105-fluorescein isothiocyanate (FITC, clone MEM-226, ImmunoTools, Friesoythe, Germany). Osteogenic, adipogenic and chondrogeriic differentiation potential of fibroblasts, MSC-AT and MSC-BM was determined as described before.

Senescence tests based on the lysosomal compartment

Expression of pH dependent senescence associated β-galactosidase (SA-β-gal) activity was simultaneously analyzed at different passages using the SA-β-gal staining kit (Cell Signaling Technology, Boston, MA) or by flow cytometry with the fluorogenic substrate 5-dodecanoylaminofluorescein di-beta-D-galactopyranoside (C12FDG). Lysosomal and mitochondrial content was analyzed upon 45 min staining of living cells with LysoTracker Red DND-99 (75 nM) and MitoTracker Green FM (100 nM, both Invitrogen/Molecular Probes, Eugene, OR, USA). Fluorescence was detected with a FACS Canto II or a Leica DM IL LED fluorescence microscope (Leica, Wetzlar, Germany).

DNA isolation and bisulfite conversion

Genomic DNA was isolated from 10$^6$ cells using the QIAGEN DNA Blood Midi-Kit. DNA quality was assessed with a NanoDrop ND-1000 spectrometer (NanoDrop Technologies, Wilmington, USA) and gel electrophoresis. 600 ng DNA were subsequently bisulfite converted using the EpiTect Bisulfite Kit (Qiagen, Hilden, Germany).

DNA methylation profiling

DNA methylation profiles were analyzed using the HumanMethylation27 Bead Chip according to the manufacturer's instructions (Illumina, San Diego, USA). During hybridization, the DNA molecules anneal to two different bead types with locus-specific DNA oligomers - one corresponds to the methylated (C) and the other to the unmethylated (T) state. Allele-specific primer annealing is followed by single-base extension using DNP-and Biotin-labeled ddNTPs. After extension, the array is fluorescently stained, scanned, and the intensities of the unmethylated and methylated bead types measured. Hybridization and initial data analysis with the BeadStudio Methylation Module were performed at the DKFZ Gene Core Facility in Heidelberg. Raw data of all hybridizations have been deposited in NCBIs Gene Expression Omnibus (GEO, http://www.ncbi.nlm.nih.gov/geo/) and are accessible through GEO Series accession numbers: GSE17448 (MSC-BM); GSE26519 (MSC-AT); GSE22595 (fibroblasts) and GSE29661 (fibroblasts and MSC-AT).

Analysis of DNA-methylation profiles

Raw data of new datasets and recently published datasets were quantile normalized to minimize chip effects. Principal components analysis (PCA) was calculated with prcomp in R package stats. For selection of relevant CpG sites we used Pavlidis Template Matching performed with the MultiExperiment Viewer (MeV, TM4.6). Therefore, templates were specified that either corresponded to 1) the passage numbers of the samples, 2) cPD or 3) days in culture. The dataset was then searched for matches to the template, based on the Pearson Correlation between the template and methylation values of the data set. For subsequent analysis we have only considered CpG sites with highly significant hyper- or hypo-methylation according to the three corresponding templates ($P < 10^{-11}$). Based on this analysis, we have selected six CpG sites - for simplicity they were termed by their corresponding genes: GRM7, CASR, PRAMEF2, SELP, CASP14 and KRTAP13-3. Methylation levels of these CpG sites were plotted against passage number for linear regression analysis with EXCEL 2007 (Microsoft). Based on these linear regressions we calculated the state of cellular aging ($N$) for each of the six CpG sites ($i$) by inserting the specific DNA-methylation levels for each gene ($\beta$).

$$N_i = (\beta_i - A_i)/B_i$$

where $A$ is the Y-axis intercept and $B$ is the slope of the corresponding CpG site in the training group. Subsequently, we determined the mean and standard deviation of the predictions of the six individual CpG sites as measure of cellular aging.

Pyrosequencing

Independent DNA samples of known passage were subsequently bisulfite converted and analyzed by pyrosequencing with regard to the six specific CpG sites. Pyrosequencing was performed at Varionostic GmbH (Ulm, Germany). Primers and sequencing primers are provided in table 1. Other primers than those disclosed herein may be used for amplifying and/or sequencing the respective nucleic acids.

Table 1: Primer for amplification of gene fragments and sequencing

|  | Gene symbol | Nucleotide sequence | SEQ ID NO: |
|---|---|---|---|
| **Forward primer** | GRM7 | TTGGGATTATTGTTGATTT | 7 |
|  | CASR | TGTAATAGGTATTTGGTTGTAGT | 8 |
|  | PRAMEF2 | TTTGAGGGTATTTAGAAGAGAT | 9 |
|  | SELP | AGAAGGTAGAAAATTAGTAGAGTT | 10 |
|  | CASP 14 | TTGGAGATTTAGTGAGATAATA | 11 |
|  | KRTAP13-3 | GAGATTTGTTGGAGGTTTAA | 12 |
| **Reverse primer** | GRM7 | CCCCTACTACCTACTAAAAATA | 13 |
|  | CASR | CCCAAACTCTTACTCATTCTA | 14 |
|  | PRAMEF2 | TCCCTAACTAACTAACTACTAATC | 15 |
|  | SELP | CAACATAAAACTCCATAACTA | 16 |
|  | CASP14 | AACAA.AACAAATAACCCATATA | 17 |
|  | KRTAP13-3 | CCCAATAAAAAACAACTCC | 18 |
| **Sequencing primer** | GRM7 | TACCTACTAAAAATACTCCT | 19 |
|  | CASR | TTGGTTGTAGTTAGGAA | 20 |
|  | PRAMEF2 | TAGAATTTTGTAAAGTGAG | 21 |
|  | SELP | AGGTAAAGGTTTAGAAAG | 22 |
|  | CASP14 | TATTTTTTTGAGATGGT | 23 |
|  | KRTAP13-3 | ATTTTTGTTTGATTATGTA | 24 |

Quantitative real-time PCR analysis

Expression of the 6 differentially methylated genes (GRM7, CASR, PRAMEF2, SELP, CASP14 and KRTAP13-3) was analyzed by quantitative real-time PCR (qRT-PCR) using the StepOne™ Instrument (Applied Biosystems [AB], Applera Deutschland GmbH, Darmstadt, Germany). Total RNA was isolated with the miRNeasy Mini Kit (Qiagen, Hilden, Germany) according to the manufacturers instructions and reversely transcribed [16]. QRT-PCR reactions were performed using Taqman® Gene Express Assays. Gene expression levels were normalized to GAPDH and 18s RNA expression.

Validation with methylation profiles

The epigenetic methylation signature was tested on published datasets of other groups. These raw data were generously provided at the public repositories GEO and Array Express. We have considered all studies with the HumanMethylation27 BeadChip. None of them provided detailed information on long-term culture. Therefore, we have used datasets of either freshly isolated cells from dermis and epidermis (E-MTAB-202 as described in Gronniger, E. et al. Aging and chronic sun exposure cause distinct epigenetic changes in human skin. PLoS. Genet. 6, e1000971 (2010)), ovarial epithelium (GSE25033 as described in Bauerschlag DO et al. Progression-Free Survival in Ovarian Cancer Is Reflected in Epigenetic DNA Methylation Profiles. Oncology 80, 12-20 (2011)), cervical smear (GSE20080 as described in Teschendorff, A. E. et al. Age-dependent DNA methylation of genes that are suppressed in stem cells is a hallmark of cancer. Genome Res. 20, 440-446 (2010)), cord blood (GSE26683 as described in Novakovic, B. et al. Wide ranging DNA methylation differences of primary trophoblast cell populations and derived-cell lines: implications and opportunities for understanding trophoblast function. Mol. Hum. Reprod. (2011)) and peripheral blood (GSE25301 as described in Wang, X. et al. Obesity related methylation changes in DNA of peripheral blood leukocytes. BMC. Med. 8, 87 (2010)) or datasets of established cell lines derived from solid cancer (cell lines SW48 and MCF-7; GSE26683 as described in Novakovic, B. et al. Wide ranging DNA methylation differences of primary trophoblast cell populations and derived-cell lines: implications and opportunities for understanding trophoblast function. Mol. Hum. Reprod (2011)), squamous cell carcinoma (GSE24091), ovarian carcinoma (GSE25033 as described in Bauerschlag, DO et al. Progression-Free Survival in Ovarian Cancer Is Reflected in Epigenetic DNA Methylation Profiles. Oncology 80, 12-20 (2011)), transformed placenta/trophoblast (GSE26683 as described in Novakovic, B. et al. Wide ranging DNA methylation differences of primary trophoblast cell populations and derived-cell lines: implications and opportunities for understanding trophoblast function. Mol. Hum. Reprod. (2011)), multiple myeloma/MGUS (GSE21304 as described in Walker, B. A. et al. Aberrant global methylation patterns affect the molecular pathogenesis and prognosis of multiple myeloma. Blood 117, 553-562 (2011)), lymphoma (GSE26133 as described in Bell, J. T. et al. DNA methylation patterns associate with genetic and gene expression variation in HapMap cell lines. Genome Biol. 12, R10 (2011)) and pluripotent cell lines (GSE24676 as described in Nishino, K. et al. DNA Methylation Dynamics in Human Induced Pluripotent Stem Cells over Time. PLoS. Genet. 7, e1002085 (2011)). For further analysis, we have only extracted beta-values of the six specific CpG-dinucleotides and used these for the predictive model described above.

The results of the analysis are briefly summarized in Figure 1. **Figure 1** illustrates that there is a clear and unequivocal correlation of the number of passages the cells went through and the methylation status of each one of the 6 CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1. GRM7-CpG-site #1 and CASR-CpG-site #1 become hypermethylated during ongoing passages of in *in-vitro* culture and thus while becoming replicative senescent, wherein the degree of hypermethylation corresponds to the number of cell passage. In contrast, the CpG-dinucleotides PRAMEF2-CpG-site #1, SELP-CpG-site #1, CASP14-CpG-site #1 and KRTAP13-3-CpG-site #1 become hypomethylated during ongoing passages of in *in-vitro* culture and thus while becoming replicative senescent, wherein the degree of hypomethylation corresponds to the number of cell passage.

**Figure 2** depicts the genomic sequences of the six CpG-dinucleotides, namely GRM7-CpG-site #1, CASR-CpG-site #1, PRAMEF2-CpG-site #1, SELP-CpG-site #1, CASP14-CpG-site #1 and KRTAP13-3-CpG-site #1, which were used for establishing the epigenetic senescence signature. Figure 2 shows the respective CpG-dinucleotides and 20 nucleotides of the genomic DNA upstream and 20 nucleotides of the genomic DNA downstream of each of said CpG-dinucleotides. The nucleotide sequences shown in Figure 2 correspond to the nucleotide sequences set forth in SEQ ID NOs: 1 to 6.

**Figure 3** shows the results where independent cell preparations were used for validation of the senescence-signature by pyrosequencing of the six CpG sites (GRM7-CpG-site #1, CASR-CpG-site #1, PRAMEF2-CpG-site #1, SELP-CpG-site #1, CASP14-CpG-site #1 and KRTAP13-3-CpG-site #1). The beta-values were used for the above mentioned linear regression models to predict the number of passages. The graph in Figure 3 clearly shows that the predicted passage number accurately identifies the real passage number of each cell culture.

**Figure 4** illustrates that the epigenetic replicative senescence signature is applicable to different cell types and tissues. DNA-methylation datasets were retrieved from public data repositories. DNA-methylation level at the six CpG-dinucleotides (GRM7-CpG-site #1, CASR-CpG-site #1, PRAMEF2-CpG-site #1, SELP-CpG-site #1, CASP14-CpG-site #1 and KRTAP13-3-CpG-site #1) can clearly separate freshly isolated cells and culture expanded cell lines (mean and standard deviation of the different samples are provided).

**Figures 5 and 6** demonstrate that CpG-dinucleotides within a region of about 50,000 bp upstream and/or downstream of at least one of the CpG-dinucleotides selected from the group consisting of GRM7-CpG-site #1 (SEQ ID No. 33), CASR-CpG-site #1 (SEQ ID No.34), PRAMEF2-CpG-site #1 (SEQ ID No. 35), SELP-CpG-site #1 (SEQ ID No. 36), CASP14-CpG-site #1 (SEQ ID No. 37) and KRTAP13-3-CpG-site #1 (SEQ ID No. 38) are all suitable to determine the senescence status of cell populations. All CpG-sites within the depicted regions are continuously methylated or demethylated with increasing senescence of the cells, i.e. a linear change of the methylation status of these CpG-dinucleotides can be observed during proceeding senescence of the cells.

The data show that the replicative senescence status of cells can be accurately determined by particular epigenetic features.

The present invention provides the advantages that it is not necessary to determine the expression of specific marker genes in order to assess the replicative senescence of a cell or the cells of a cell culture. Moreover, comparing the methylation status obtained with a reference methylation status can be done in that the methylation status as determined is inserted into a formula for linear regression. Thereby, it is possible to determine the replicative senescence status of a cell or of the cells within a cell culture, even if no positive or negative control is available. In addition, the methods of the present invention are reliable as the results have been proven to be independent from the persons handling the cell cultures, any growth conditions and the like.

EXAMPLE 2

In this approach, all available DNAm data with the Infinium HumanMethylation27 BeadChip were combined. This platform facilitates simultaneous analysis of DNAm at more than 27,000 CpG sites at single base resolution. Subsequently, those CpG sites which reveal a linear increase in DNAm level over either subsequent passages, days in culture or cumulative population doublings (cPDs) have been identified and selected. Six specific CpG sites were identified by Pavlidis template matching which fulfilled these parameters. Analysis of DNAm at these CpG sites can conversely be used to predict the state of cellular aging. In the following sections, a step-by-step guidance for the usage of this Epigenetic Senescence Signature is provided.

**Material**

Biological Material

This method is applicable for different cell types - particularly for mesenchymal stromal cells (MSC) and fibroblasts. All cell preparations were isolated after written consent according to the guidelines of the local Ethics Committes. Bone marrow derived MSC (BM-MSC) were isolated from bone marrow aspirates (iliac crest; IC) of healthy donors for allogeneic transplantation (#348/2004, Heidelberg, Germany), from caput femoris (CF) after hip fracture or from tibia plateau (TP; #EK128/09, Aachen, Germany, and #076/2007, Heidelberg). Adipose-tissue derived MSC (AT-MSC) were isolated from lipoaspirates of healthy adult donors (#EK163/07, Aachen). Dermal fibroblasts (D-Fib) were isolated from patients undergoing plastic surgery (#EK173/07, Aachen). The following sections describe exemplarily culture expansion of BM-MSC in medium supplemented with 10% human platelet lysate (hPL).

Human platelet lysate

Human platelet lysate (hPL) is generated by simple freeze-thaw procedures with thrombocyte concentrate units from healthy donors (provided by the blood bank, University Hospital, Aachen).

Culture Medium and Passaging

1. 500 mL laboratory bottle (Duran, Wertheim, Germany)
1. 500 mL rapid-Filtermax, vacuum filtration unit (TPP, Trasadingen, Switzerland)
1. 2 U/mL Heparin (Ratiopharm, Ulm, Germany)
2. Dulbecco's Modified Eagles Medium-Low Glucose (DMEM-LG; PAA, Pasching, Austria)
3. 2 mM L-glutamine (Sigma-Aldrich, München, Germany)

4. 100 U/mL penicillin/streptomycin (pen/strep; Gibco, Invitrogen, Carlsbad, USA)
5. 0.25% Trypsin-EDTA solution (1x) (Gibco)
6. 1x phosphate-buffered saline (PBS) (PAA)
7. Tissue culture flasks 75 cm2 (Nunc Thermo Fisher Scientific, Langenselbold, Germany)
8. Hemocytometer (Neubauer counting chamber, Brand, Wertheim, Germany)
9. 0.4% Trypan Blue solution (Sigma)
10. 15 and 50 mL Falcon tubes (BD)

DNA preparation

1. QiaAmp DNA Blood Midi Kit (Qiagen, Hilden Germany)
2. 15 mL Falcon tubes (BD)
3. 0.5 mL Safelock tubes (Sarstedt, Nümbrecht, Germany)
4. Nuclease-Free Water (Qiagen)
5. Waterbath (70°C)

DNAm analysis at specific CpG sites

This method is based on the DNAm level at six specific CpG sites. Various methods can be used for this purpose. These CpG sites can easily be selected from DNAm profiles with the Infinium HumanMethylation27 BeadChip. It is expected that it is alternatively possible to determine the DNAm level by MassARRAY assay. In the following sections the specific analysis of pyrosequencing of bisulfide-treated DNA is used.

**Methods**

Long-term culture of mesenchymal stomal cells

1. Add L-glutamine, penicillin/streptomycin, heparin and human platelet lysate (10%) to the DMEM-LG medium to a final volume of 500 mL.
2. Sterile-filter medium and store at 4°C up to 2 weeks.
3. Seed BM-MSC after isolation at a density of 5,000 cells/cm$^2$ into culture flasks.
4. Upon 80% confluence remove medium and wash cells with PBS twice.
5. Add 1 mL of trypsin-EDTA solution and incubate at 37°C for 1 minute.
6. After cell detachment, stop trypsination by application of 5 mL culture medium.
7. Transfer solution into 15 mL Falcon tube and centrifuge at 350 x g for 7 minutes.
8. Discard supernatant and resuspend pellet in 1mL culture medium.
9. Count cells in a Neubauer counting chamber after application of Trypan Blue dye for exclusion of dead cells.
10. Re-seed cells at a density of 5,000 cells/cm$^2$.
11. Document cell count for calculation of cumulative Population Doublings (see subheading 3.3).

Calculation of real cumulative population doublings

1. Calculate cumulative Population Doublings from the first passage until the destined number of passages by employing the following formula **(Figure 7):**

$$cPD = sum_{i=1...n} \log_2(h_i/s_i),$$

where n is the total number of passages; si is the number of cells seeded at passage i and h$i$ is the number of cells harvested in passage i.

DNA preparation and bisulfide conversion

1. Isolate genomic DNA with the QiaAmp DNA Blood Midi Kit following the manufacturer's instructions.
2. Elute DNA in 300 μL Nuclease-Free Water or in the provided Elution Buffer for long-term storage.
3. Assess concentration and quality via photometric measurement and agarose gel electrophoresis.
4. Bisulfide conversion

DNA methylation analysis of the 6 specific CpG sites

Analysis of the DNAm 6 specific CpG sites is the prerequisite for usage of the Epigenetic Senescence Signature. If DNAm profiles with the Illumina HumanMethylation27k Chip are available these can be directly extracted by the IDs (corresponding gene names and sequences are also indicated - the relevant CpG sites are indicated bold):

1. GTGCTGGAGGTGCTCCTGTG**CG**CGCTGGCGGCGGCGGCGCGC (cg02332525; GRM7)
2. TGGCTGCAGCCAGGAAGGAC**CG**CACGCCCTTTCGCGCAGGAG (cg17453778; CASR)
3. AGAAGGTGGTGACTTACCAG**CG**CTGGACTCACTTTGCAGAGT (cg03891191; PRAMEF2)
4. ACATAAAACTCCATGGCTAT**CG**CTGTTCCTCACTTTCTGAAC (cg01459453; SELP)
5. CTCTTCTACCTAGGAGATGA**CG**GGCTGGGGAAGCCATCTCAA (cg01999333; CASP14)
6. TGACTATGCATGTTGGGTCT**CG**GGGTTTTGGATCCAATAGCT (cg16431978; KRTAP13-3)

Alternatively, DNAm can be specifically determined by pyrosequencing as described above.

Analysis of the Epigenetic Senescence Signature with a computer-readable program

For each CpG site the corresponding DNAm values need to be inserted in the corresponding linear regression models **(Figure 8).** These equations are described in Koch et al. (2012), Aging Cell 11,366-9. Thereby, predictions for cPDs are generated for each individual CpG site and the mean of these is subsequently calculated for the final value **(Figure 9).** To make this calculation easier a software was designed, where the DNAm values can be easily integrated. The input mask of this software is shown in **Figure 10.**

1. Type in the corresponding beta-values of the 6 CpG sites and press *Calculate* for the predictions.
2. Compare results with the calculated real cPD of the cell preparations.

Alternatively, a multivariate model is provided, which combines the six linear regressions into one equation:

$$\text{Predicted cPD} = 45.89 + (23.63 * \text{cg02332525}) + (31.61 * \text{cg17453778}) + (-53.70 * \text{cg03891191}) + (14.86 * \text{cg01459453}) + (-23.94 * \text{cg01999333}) + (-10.34 * \text{cg16431978})$$

The corresponding beta-values for the six CpG sites have to be inserted. This method generates similar results as the above mentioned method (Figure 10).

**Claims**

1. A quantitative method for determining the replicative senescence status of a cell propagated in an *in-vitro* culture, the method comprising the steps of:

   a) determining the methylation status of at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 and
   b) comparing the Methylation status of each of the at least one CpG-dinucleotides investigated with a reference methylation status of the respective CpG-dinucleotide,

   wherein the methylation status of said CpG-dinucleotide linearly correlates with the replicative senescence status of said cell, thereby determining the approximate time, said cells have been in culture, and/or the approximate passage number of said cells and/or the number of population doublings.

2. The method according to claim 1, wherein the methylation status of at least one of the CpG-dinucleotides within a region of about 5,000 bp upstream and/or downstream, preferably of about 3,000 bp upstream and/or downstream, more preferably of about 1,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 is determined.

3. The method according to claim 1 or 2, wherein the replicative senescence status is determined in that the methylation status is determined for at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 for multiple corresponding DNA molecules.

4. The method according to any one of claims 1 to 3, wherein the cell is selected from the group consisting of stromal cells and induced pluripotent stem cells.

5. The method according to any one of claims 1 to 4, wherein the methylation status of two, three, four, five or all of said six CpG-dinucleotides from different DNA molecules which are selected from the group consisting of DNA molecules comprising at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1 is determined.

6. The method according to any one of claims 1 to 5, wherein the methylation status is determined by one or more methods selected from the group consisting of methylation specific PCR, COBRA-Assay, methylation-specific restriction pattern analysis, CHIP-sequencing, methyl-CAP-sequencing, and preferably sequence analysis of bisulfite-treated DNA.

7. Use of at least one nucleic acid molecule for determining the replicative senescence status of a cell according to the method of any one of claims 1 to 6, wherein said nucleic acid molecule comprises at least one nucleotide sequence selected from the group consisting of:

   a) a nucleotide sequence comprising at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1,
   b) a nucleotide sequence which differs from the nucleotide sequence of a) by replacement of at most 10 % of the nucleotides, but for said CpG-dinucleotide, and
   c) a nucleotide sequence which corresponds to the complementary strand of the nucleotide sequence of a) or b).

8. Use of a kit in the method of any one of claims 1 to 6, the kit comprises at least one set of oligonucleotide primers for amplifying and/or analyzing at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

9. The use according to claim 8, wherein the kit comprises a pair of oligonucleotide primers for amplifying and/or at least one oligonucleotide primer for analyzing at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1.

10. The use according to claim 8 or 9, wherein the kit further comprises at least one reaction buffer and/or reagents for at least one method selected from the group consisting of PCR-amplification, bisulfite-conversion of DNA, DNA-sequencing, preferably DNA-pyrosequencing, and COBRA-assay.

11. Computer-readable medium having stored computer-executable instructions for causing a computer to perform a method for determining the replicative senescence status of a cell according to the method of any one of claims 1 to 6 comprising:

- inputting at least one value of the determined methylation status of at least one of the CpG-dinucleotides within a region of about 10,000 bp upstream and/or downstream of at least one of the CpG-dinucleotides selected from the group consisting of GRM7-CpG-site #1, CASR-CpG-site #1, PRAMEF2-CpG-site #1, SELP-CpG-site #1, CASP14-CpG-site #1 and KRTAP13-3-CpG-site #1,
- comparing said value of the determined methylation status with stored data representing a correlation between the methylation status of said CpG-dinucleotide and the replicative senescence status of the cell, and
- displaying the replicative senescence status of the cell.

**12.** Computer-readable medium according to claim 11, wherein said stored data comprise at least one linear regression equation.

**Patentansprüche**

**1.** Quantitatives Verfahren zur Bestimmung des Status der replikativen Seneszenz einer in *in-vitro* - Kultur vermehrten Zelle, wobei das Verfahren die folgenden Schritte umfasst:

a) Bestimmen des Methylierungsgrades von mindestens einem der CpG-Dinukleotide innerhalb einer Region von ungefähr 10.000 bp stromaufwärts und/oder stromabwärts von mindestens einem der CpG-Dinukleotide, die aus der Gruppe bestehend aus GRM7-CpG-Position #1, CASR-CpG- Position #1, PRAMEF2-CpG- Position #1, SELP-CpG- Position #1, CASP14-CpG- Position #1 und KRTAP13-3-CpG- Position #1 ausgewählt sind, und
b) Vergleichen des Methylierungsgrades von jedem der mindestens einem untersuchten CpG-Dinukleotide mit einem Referenz-Methylierungsgrad des jeweiligen CpG-Dinukleotids,

wobei der Methylierungsgrad des CpG-Dinukleotids mit dem Status der replikativen Seneszenz der Zelle linear korreliert, wodurch die ungefähre Dauer, für die sich die Zellen in Kultur befunden haben, und/oder die ungefähre Anzahl an Zellpassagen und/oder die Anzahl der Populationsverdopplungen bestimmt wird.

**2.** Verfahren nach Anspruch 1, wobei der Methylierungsgrad von mindestens einem der CpG-Dinukleotide innerhalb einer Region von ungefähr 5.000 bp stromaufwärts und/oder stromabwärts, bevorzugt ungefähr 3.000 bp stromaufwärts und/oder stromabwärts, mehr bevorzugt ungefähr 1.000 bp stromaufwärts und/oder stromabwärts von mindestens einem der CpG-Dinukleotide, die aus der Gruppe bestehend aus GRM7-CpG- Position #1, CASR-CpG-Position #1, PRAMEF2-CpG-Position #1, SELP-CpG- Position #1, CASP14-CpG- Position #1 und KRTAP13-3-CpG- Position #1 ausgewählt sind, bestimmt wird.

**3.** Verfahren nach Anspruch 1 oder 2, wobei der Status der replikativen Seneszenz dadurch bestimmt wird, dass der Methylierungsgrad für mindestens eines der CpG-Dinukleotide, die aus der Gruppe bestehend aus GRM7-CpG-Position #1, CASR-CpG- Position #1, PRAMEF2-CpG-Position #1, SELP-CpG- Position #1, CASP14-CpG- Position #1 und KRTAP13-3-CpG- Position #1 ausgewählt sind, für mehrere dazugehörige DNA Moleküle bestimmt wird.

**4.** Verfahren nach einem der Ansprüche 1 bis 3, wobei die Zelle aus der Gruppe bestehend aus Stroma-Zellen und induzierten pluripotenten Stammzellen ausgewählt ist.

**5.** Verfahren nach einem der Ansprüche 1 bis 4, wobei der Methylierungsgrad von zwei, drei, vier, fünf oder allen der sechs CpG-Dinukleotide verschiedener DNA Moleküle, die aus der Gruppe bestehend aus DNA-Molekülen, welche mindestens eines der CpG-Dinukleotide umfassen, die aus der Gruppe bestehend aus GRM7-CpG-Position #1, CASR-CpG- Position #1, PRAMEF2-CpG- Position #1, SELP-CpG- Position #1, CASP14-CpG- Position #1 und KRTAP13-3-CpG- Position #1 ausgewählt sind, bestimmt wird.

**6.** Verfahren nach einem der Ansprüche 1 bis 5, wobei der Methylierungsgrad durch ein oder mehrere Verfahren bestimmt wird, welches aus der Gruppe bestehend aus Methylierungs-spezifischer PCR, COBRA-Assay, Methylierungs-spezifischer Restriktionsmusteranalyse, CHIP-Sequenzierung, Methyl-CAP-Sequenzierung und vorzugsweise Sequenz-Analyse von Bisulfitbehandelter DNA ausgewählt ist.

**7.** Verwendung mindestens eines Nukleinsäure-Moleküls zur Bestimmung des Status der replikativen Seneszenz einer Zelle gemäß dem Verfahren nach einem der Ansprüche 1 bis 6, wobei das Nukleinsäure-Molekül mindestens eine Nukleotid-Sequenz umfasst, die ausgewählt ist aus der Gruppe bestehend aus:

a) einer Nukleotid-Sequenz, die mindestens eines der CpG-Dinukleotide innerhalb einer Region von 10.000 bp stromaufwärts und/oder stromabwärts von mindestens einem der CpG-Dinukleotide, die aus der Gruppe bestehend aus GRM7-CpG-Position #1, CASR-CpG-Position #1, PRAMEF2-CpG- Position #1, SELP-CpG- Position #1, CASP14-CpG- Position #1 und KRTAP13-3-CpG- Position #1 ausgewählt sind, umfasst,

b) einer Nukleotid-Sequenz, welche sich mit Ausnahme des CpG-Dinukleotids von der Nukleotid-Sequenz gemäß a) durch den Austausch von höchstens 10 % der Nukleotide unterscheidet, und

c) einer Nukleotid-Sequenz, welche dem komplementären Strang der Nukleotid-Sequenz gemäß a) oder b) entspricht.

**8.** Verwendung eines Kits im Verfahren nach einem der Ansprüche 1 bis 6, wobei das Kit mindestens einen Satz von Oligonukleotid-Primern zum Amplifizieren und/oder Analysieren mindestens eines der CpG-Dinukleotide innerhalb einer Region von 10.000 bp stromaufwärts und/oder stromabwärts von mindestens einem der CpG-Dinukleotide, die aus der Gruppe bestehend aus GRM7-CpG-Position #1, CASR-CpG-Position #1, PRAMEF2-CpG- Position #1, SELP-CpG- Position #1, CASP14-CpG- Position #1 und KRTAP13-3-CpG- Position #1 ausgewählt sind, umfasst.

**9.** Verwendung nach Anspruch 8, wobei das Kit ein Paar von Oligonukleotid-Primern zum Amplifizieren und/oder mindestens einen Oligonukleotid-Primer zum Analysieren von mindestens einem der CpG-Dinukleotide, die aus der Gruppe bestehend aus GRM7-CpG-Position #1, CASR-CpG- Position #1, PRAMEF2-CpG- Position #1, SELP-CpG-Position #1, CASP14-CpG- Position #1 und KRTAP13-3-CpG- Position #1 ausgewählt sind, umfasst.

**10.** Verwendung nach Anspruch 8 oder 9, wobei das Kit ferner mindestens einen Reaktionspuffer und/oder Reagenzien für mindestens ein Verfahren, das aus der Gruppe bestehend aus PCR-Amplifikation, Bisulfit-Umwandlung von DNA, DNA-Sequenzierung, bevorzugt DNA-Pyrosequenzierung und COBRA-Assay ausgewählt ist, umfasst.

**11.** Computer-lesbares Medium mit darauf gespeicherten, auf einem Computer ausführbaren Anweisungen zum Veranlassen eines Computers zur Ausführung des Verfahrens zur Bestimmung des Status der replikativen Seneszenz einer Zelle gemäß dem Verfahren nach einem der Ansprüche 1 bis 6 umfassend:

- Eingeben mindestens eines Wertes des ermittelten Methylierungsgrads mindestens eines der CpG-Dinukleotide innerhalb einer Region von 10.000 bp stromaufwärts und/oder stromabwärts von mindestens einem der CpG-Dinukleotide, die aus der Gruppe bestehend aus GRM7-CpG-Position #1, CASR-CpG-Position #1, PRAMEF2-CpG- Position #1, SELP-CpG- Position #1, CASP14-CpG- Position #1 und KRTAP13-3-CpG- Position #1 ausgewählt sind,

- Vergleichen dieses Wertes des ermittelten Methylierungsgrads mit gespeicherten Daten, die eine Korrelation zwischen dem Methylierungsgrad des CpG-Dinukleotids und der replikativen Seneszenz der Zelle repräsentieren, und

- Anzeigen des Status der replikativen Seneszenz der Zelle.

**12.** Computer-lesbares Medium nach Anspruch 11, wobei die gespeicherten Daten mindestens eine lineare Regressionsgleichung umfassen.

**Revendications**

**1.** Procédé quantitatif pour déterminer l'état de sénescence réplicative d'une cellule propagée dans une culture in vitro, le procédé comprenant les étapes consistant à :

a) déterminer l'état de méthylation d'au moins un des dinucléotides CpG à l'intérieur d'une région d'environ 10 000 pb en amont et/ou en aval d'au moins un des dinucléotides CpG choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1 et

b) comparer l'état de méthylation de chacun de l'au moins un dinucléotide CpG sous investigation avec un état de méthylation de référence du dinucléotide CpG respectif,

dans lequel le statut de méthylation dudit dinucléotide CpG est en corrélation de manière linéaire avec l'état de sénescence réplicative de ladite cellule, déterminant ainsi le temps approximatif, lesdites cellules ont été mises en culture, et/ou le nombre de passages approximatifs desdites cellules et/ou le nombre de dédoublements de la population.

**2.** Procédé selon la revendication 1, dans lequel l'état de méthylation d'au moins un des dinucléotides CpG à l'intérieur d'une région d'environ 5 000 pb en amont et/ou en aval, de préférence d'environ 3 000 pb en amont et/ou en aval, encore plus préférablement d'environ 1 000 pb en amont et/ou en aval d'au moins un des dinucléotides CpG choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1 est déterminé.

**3.** Procédé selon la revendication 1 ou la revendication 2, dans lequel l'état de sénescence réplicative est déterminé en ce que l'état de méthylation d'au moins un des dinucléotides CpG choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1 pour de multiples molécules d'ADN correspondantes.

**4.** Procédé selon l'une quelconque des revendications 1 à 3, dans lequel la cellule est choisie dans le groupe constitué des cellules stromales et des cellule souches pluripotentes induites.

**5.** Procédé selon l'une quelconque des revendications 1 à 4, dans lequel l'état de méthylation de deux, trois, quatre, cinq ou de tous lesdits six dinucléotides CpG à partir de molécules d'ADN différentes qui sont choisies dans le groupe constitué des molécules d'ADN comprenant au moins un des dinucléotides choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1 est déterminé.

**6.** Procédé selon l'une quelconque des revendications 1 à 5, dans lequel l'état de méthylation est déterminé par un ou plusieurs procédés choisis dans le groupe constitué par la PCR spécifique de la méthylation, l'essai COBRA, l'analyse de motifs de restriction spécifique de la méthylation, le séquençage CHIP, le séquençage méthyl-CAP, et de préférence l'analyse séquentielle d'ADN traité au bisulfite.

**7.** Utilisation d'au moins une molécule d'acide nucléique pour déterminer l'état de sénescence réplicative d'une cellule selon le procédé de l'une quelconque des revendications 1 à 6, dans laquelle ladite molécule d'acide nucléique comprend au moins une séquence nucléotidique choisie dans le groupe constitué de :

a) une séquence nucléotidique comprenant au moins un des dinucléotides CpG à l'intérieur d'une région d'environ 10 000 pb en amont et/ou en aval d'au moins un des dinucléotides CpG choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1,

b) une séquence nucléotidique qui diffère de la séquence nucléotidique de a) par le remplacement de pas plus de 10 % des nucléotides, mais pour ledit dinucléotide CpG, et

c) une séquence nucléotidique qui correspond au brin complémentaire de la séquence nucléotidique de a) ou de b).

**8.** Utilisation d'un kit dans le procédé selon l'une quelconque des revendications 1 à 6, le kit comprend au moins un ensemble d'amorces oligonucléotidiques pour l'amplification et/ou l'analyse d'au moins un des dinucléotides CpG à l'intérieur d'une région d'environ 10 000 pb en amont et/ou en aval d'au moins un des dinucléotides CpG choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1.

**9.** Utilisation selon la revendication 8, dans laquelle le kit comprend une paire d'amorces oligonucléotidiques pour l'amplification et/ou, au moins une amorce oligonucléotidique pour l'analyse d'au moins un des dinucléotides CpG choisi dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1.

**10.** Utilisation selon la revendication 8 ou la revendication 9, dans laquelle le kit comprend en outre au moins un tampon de réaction et/ou des réactifs pour au moins un procédé choisi dans le groupe constitué par l'amplification par PCR, la conversion bisulfite d'ADN, le séquençage d'ADN, de préférence le pyroséquençage d'ADN, et l'essai COBRA.

**11.** Support lisible par ordinateur possédant des instructions stockées exécutables par ordinateur pour faire en sorte qu'un ordinateur effectue un procédé pour déterminer l'état de sénescence réplicative d'une cellule selon le procédé de l'une quelconque des revendications 1 à 6 consistant à :

- saisir au moins une valeur de l'état de méthylation déterminé d'au moins un des dinucléotides CpG à l'intérieur d'une région d'environ 10 000 pb en amont et/ou en aval d'au moins un des dinucléotides CpG choisis dans le groupe constitué par le site GRM7-CpG #1, le site CASR-CpG #1, le site PRAMEF2-CpG #1, le site SELP-CpG #1, le site CASP14-CpG #1 et le site KRTAP13-3-CpG #1,
- comparer ladite valeur de l'état de méthylation déterminé avec une valeur stockée représentant une corrélation entre l'état de méthylation dudit dinucléotide et l'état de sénescence réplicative de la cellule, et
- afficher l'état de sénescence réplicative de la cellule.

**12.** Support lisible par ordinateur selon la revendication 11, dans lequel ladite donnée stockée comprend au moins une équation de régression linéaire.

# Fig. 1

# Fig. 2

| | |
|---|---|
| **GRM7** | GTGCTGGAGGTGCTCCTGTGCGCGCTGGCGGCGGCGGCGCGC |
| **CASR** | TGGCTGCAGCCAGGAAGGACCGCACGCCCTTTCGCGCAGGAG |
| **PRAMEF2** | AGAAGGTGGTGACTTACCAGCGCTGGACTCACTTTGCAGAGT |
| **SELP** | ACATAAAACTCCATGGCTATCGCTGTTCCTCACTTTCTGAAC |
| **CASP14** | CTCTTCTACCTAGGAGATGACGGGCTGGGGAAGCCATCTCAA |
| **KRTAP13-3** | TGACTATGCATGTTGGGTCTCGGGGTTTTGGATCCAATAGCT |

# Fig. 3

**Fig. 4**

cell lines:
lymphoblastoid cell lines; GSE26133
multiple myeloma/MGUS cell lines; GSE21304
placenta/trophobl. cell lines; GSE26683
ovarian carcinoma cell line; GSE25033
squamous cell carcinoma lines; GSE24091
solid cancer cell lines; GSE26683

primary cells:
peripherial blood; GSE25301
cord blood; GSE26683
cervical smear cells; GSE20080
ovarial epithelium; GSE25033
epidermis; E-MTAB-202
dermis; E-MTAB-202

pluripotent cell lines:
IPS; GSE24676
ESC; GSE24676

predicted passage (based on 6 CpGs)

# FIG. 5

# FIG. 6

# Fig. 7

# Fig. 8

A

CpG1, CpG2, CpG3, CpG4, CpG5, CpG6

B

CpG1, CpG2, CpG3, CpG4, CpG5, CpG6

cumulative Population Doublings

cumulative Population Doublings

▲ D-Fib; 10% FCS
☐ AT-MSC; 10% FCS
■ AT-MSC; 10% hPL
○ BM-MSC (CF); 2% FCS; PDGF; EGF
● BM-MSC (IC); 2% FCS; PDGF; EGF
✿ BM-MSC (TP); 10% hPL

# Fig. 9

A

real cPD vs predicted cPD

B

real passages vs predicted passages

C

real days in culture vs predicted days in culture

▲ D-Fib; 10% FCS
■ AT-MSC; 10% hPL
◉ BM-MSC (TP); 10% hPL

# Fig. 10

## Monitoring of Cellular Senescence by DNA-Methylation at Specific CpG sites

Carmen M. Koch, Sylvia Joussen, Anne Schellenberg, Qiong Lin, Martin Zenke, Wolfgang Wagner

**Instruction for Web Tool:**

1. Prerequisite for this tool is the detection of the methylation levels of CpG sites highlighted in red, e.g. by pyrosequencing or Illumina Human Methylation BeadChip.

2. Enter methylation levels into the corresponding fields below (such as beta values, ranging from 0 to 100%).

3. Press the "Calculate" button and retrieve the predicted condition of the cell preparation.

| | Gene | Sequence | Methylation level % |
|---|---|---|---|
| CpG1 | GRM7 | GTGCTGGAGGTGCTCCTGTGCGCGCTGGCGGCGGCGGCGCGC | 4 |
| CpG2 | CASR | TGGCTGCAGCCAGGAAGGACCGCACGCCCTTTCGCGCAGGAG | 30 |
| CpG3 | PRAMEF2 | AGAAGGTGGTGACTTACCAGCGCTGGACTCACTTTGCAGAGT | 30 |
| CpG4 | SELP | ACATAAAACTCCATGGCTATCGCTGTTCCTCACTTTCTGAAC | 46 |
| CpG5 | CASP14 | CTCTTCTACCTAGGAGATGACGGGCTGGGGAAGCCATCTCAA | 23 |
| CpG6 | KRTAP13-3 | TGACTATGCATGTTGGGTCTCGGGGTTTTGGATCCAATAGCT | 16 |

[ Calculate ]

predicted passage: *19*

predicted cumulative population doublings: *40*

predicted time in culture: *109*

[ Start All Over ]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BORK, S. et al.** DNA Methylation Pattern Changes upon Long-Term Culture and Aging of Human Mesenchymal Stromal Cells. *Aging Cell,* 2010, vol. 9, 54-63
- **KOCH, C. et al.** Specific Age-associated DNA Methylation Changes in Human Dermal Fibroblasts. *PLoS ONE,* 2011, vol. 6, e16679
- **GRONNIGER, E. et al.** Aging and chronic sun exposure cause distinct epigenetic changes in human skin. *PLoS. Genet.,* 2010, vol. 6, e1000971
- **BAUERSCHLAG DO et al.** Progression-Free Survival in Ovarian Cancer Is Reflected in Epigenetic DNA Methylation Profiles. *Oncology,* 2011, vol. 80, 12-20
- **TESCHENDORFF, A. E. et al.** Age-dependent DNA methylation of genes that are suppressed in stem cells is a hallmark of cancer. *Genome Res.,* 2010, vol. 20, 440-446
- **NOVAKOVIC, B. et al.** Wide ranging DNA methylation differences of primary trophoblast cell populations and derived-cell lines: implications and opportunities for understanding trophoblast function. *Mol. Hum. Reprod.,* 2011

- **WANG, X. et al.** Obesity related methylation changes in DNA of peripheral blood leukocytes. *BMC. Med.,* 2010, vol. 8, 87
- **NOVAKOVIC, B. et al.** Wide ranging DNA methylation differences of primary trophoblast cell populations and derived-cell lines: implications and opportunities for understanding trophoblast function. *Mol. Hum. Reprod,* 2011
- **BAUERSCHLAG, DO et al.** Progression-Free Survival in Ovarian Cancer Is Reflected in Epigenetic DNA Methylation Profiles. *Oncology,* 2011, vol. 80, 12-20
- **WALKER, B. A. et al.** Aberrant global methylation patterns affect the molecular pathogenesis and prognosis of multiple myeloma. *Blood,* 2011, vol. 117, 553-562
- **BELL, J. T. et al.** DNA methylation patterns associate with genetic and gene expression variation in HapMap cell lines. *Genome Biol.,* 2011, vol. 12, R10
- **NISHINO, K. et al.** DNA Methylation Dynamics in Human Induced Pluripotent Stem Cells over Time. *PLoS. Genet.,* 2011, vol. 7, e1002085
- **KOCH et al.** *Aging Cell,* 2012, vol. 11, 366-9